# EUROPEAN PATENT APPLICATION

(11) **EP 4 650 809 A1**
(43) Date of publication of application: **19.11.2025**
(21) Application number: 25176733.1
(22) Date of filing: 15.05.2025
(51) Int. Cl.: G01R 33/54, A61B 5/00, G01R 33/565

(54) **MAGNETIC RESONANCE IMAGING APPARATUS AND DISPLAY METHOD FOR PRESENTING SCAN INFORMATION AND BODY MOVEMENT INFORMATION TO USER**

(30) Priority: 15.05.2024 JP 2024079654
(71) Applicant: FUJI-FILM Corporation, Minato-ku Tokyo 106-8620 (JP)
(72) Inventor: IKEGAWA, Ayaka, Tokyo, 106-8620 (JP); HAMADA, Kota, Tokyo, 106-8620 (JP)
(74) Representative: Dehns Germany Partnerschaft mbB

(57) **Abstract**

For a scan task in which a plurality of cross-sectional images that are related to each other in imaging positions are imaged, a relationship between scans and body movements is presented to a user in an easily understandable manner.

In a case where imaging including a plurality of scan tasks of cross sections is performed, progress of a scan and body movement information including a magnitude and an occurrence time of a body movement collected by a body movement processing unit are associated with each other, and on a display screen of a display device (30) having a display region set to display at least one of the plurality of scan tasks, an image obtained in each scan, and the body movement information, the body movement information is displayed in association with the progress of the scan in the display region of the at least one.

## Description

### CROSS-REFERENCE TO RELATED APPLICATIONS

The present application claims priority to Japanese Patent Application No. 2024-079654, filed May 15, 2024.

### BACKGROUND OF THE INVENTION

### 1. Field of the Invention

The present invention relates to an image diagnostic apparatus such as a magnetic resonance imaging apparatus (hereinafter, referred to as an MRI apparatus), and particularly to a user interface (UI) technology that presents scan information in progress and body movement information of a subject occurring during a scan.

### 2. Description of the Related Art

In imaging using an image diagnostic apparatus such as an MRI apparatus, a movement of a subject during imaging causes a significant deterioration in image quality, and thus processing of a body movement is an important issue. Therefore, a technology (body movement correction technology) of monitoring a body movement of a subject during imaging and correcting an image obtained by the imaging using body movement information obtained by the monitoring is widely used. In the monitoring of the body movement, a surveillance camera or an optical detection device using infrared light or the like is installed in the vicinity of the image diagnostic apparatus or in an imaging space, a temporal change in body posture of the subject is detected based on a video or a signal obtained from the device, and the image diagnostic apparatus imports the temporal change as body movement information and corrects data acquired by the imaging or discards and re-measures the data, thereby obtaining images in which effects of the body movement are suppressed.

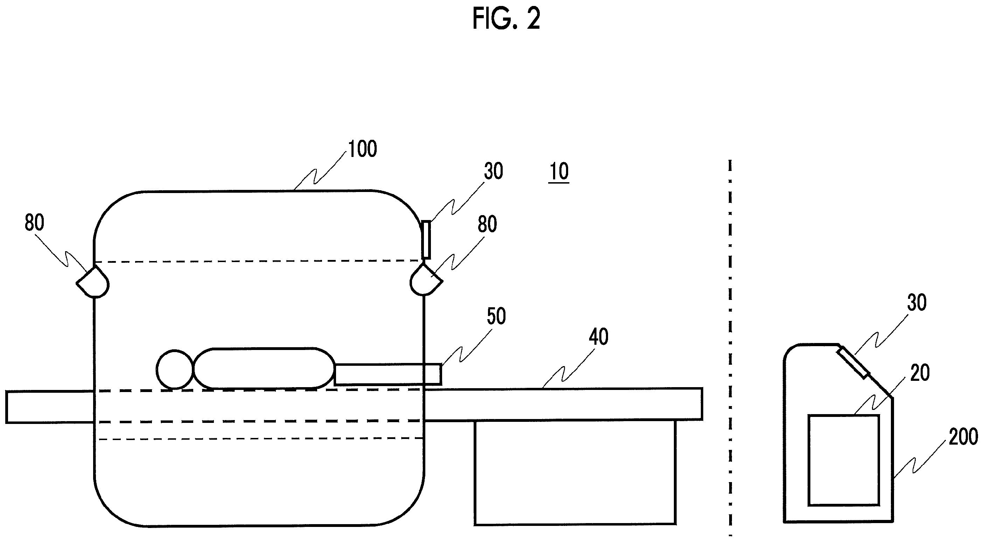

However, since the correction of measurement data using the body movement information, that is, body movement correction is performed as internal processing of the image diagnostic apparatus, it is not possible for a doctor or an examination technician involved in the imaging (hereinafter, collectively referred to as a user) to know in real time at what point in time during imaging the body movement that has a large effect on image quality occurs. For this problem, for example, JP2015-198958A discloses that a marker indicating that a subject has moved by a predetermined threshold or more is displayed on a list of scans (pulse sequences) included in a protocol or on a scan image. In addition, WO2017/102860A discloses a monitoring device that tracks movement of a subject during a scan, describes graphical representation of a displacement of the subject along with an image obtained by the scan and displaying of an indicator indicating the start or the end of the scan on the graph (claim 6, Fig. 2, and the like).

### SUMMARY OF THE INVENTION

In a body movement display method in the related art, scan information and body movement information are displayed in association with each other, allowing a grasp of which scan has been affected by a body movement. However, for example, in the technology described in JP2015-198958A, it is only possible to determine whether or not the body movement equal to or greater than a predetermined threshold has occurred, and it is not possible to identify whether or not the body movement affects a plurality of scans that are related to each other. In the technology described in WO2017/102860A, a magnitude of the body movement during a scan can be known by displaying the body movement in a graph, but similar problems are also incurred.

In addition, it is common for MRI to acquire images of a plurality of cross sections such as an axial image (AX image), a sagittal image (SAG image) plane, and a coronal image (COR image) for the same imaging area, and diagnosis is performed with reference to the images of the plurality of cross sections. In that case, in a case where a body movement occurs during scanning any cross section, artifacts, positional deviations, or the like may occur, which may impede diagnosis. This problem also applies to a case where a positioning image for determining an imaging cross section position for main imaging or images of a plurality of cross sections for adjusting scan parameters (FOV, the number of slices, and the like) for the main imaging are acquired. For example, in a case of determining a position of another cross section based on one cross-sectional image, in a case where there is a body movement during imaging of the cross-sectional image serving as a base, a positional deviation or the like may occur in the next cross section determined based on the cross-sectional image in which the body movement has occurred, and the next cross section may not be specified appropriately. As described above, in the related art, only displaying information of a body movement in association with each independent scan is suggested, and there is no display that allows a user to know at a glance which image has body movement for a plurality of images having a relationship with each other.

An aspect of the present invention provides a unit that presents, for a scan task in which a plurality of cross-sectional images that are related to each other in imaging positions are imaged, a relationship between scans and body movements to a user in an easily understandable manner.

In order to solve the above problems, according to the aspect of the present invention, in a case where continuous scan tasks for imaging a plurality of cross sections are performed, progress (time element) of each of the scan tasks and body movement information (time element and spatial element) acquired in the progress of the scan tasks are associated with each other, and information associated in real time with the progress of the scan task is presented, thereby enabling a user to quickly judge whether or not the continuous scan tasks are appropriate and to respond based on the determination.

That is, an MRI apparatus according to an aspect of the present invention comprises: an imaging unit that performs a plurality of scan tasks including imaging of different cross sections and collects a nuclear magnetic resonance signal generated from a subject for each of scans; and a processor including an image generation unit that generates an image of each of the scans using the nuclear magnetic resonance signal, a body movement processing unit that collects and processes body movement information related to a body movement of the subject, and a display control unit that displays the image and the body movement information on a display device. The processor associates progress of the scan with the body movement information including a magnitude and an occurrence time of the body movement collected by the body movement processing unit, and the display control unit sets a display region on the display device to display at least one of the plurality of scan tasks, the image obtained in each of the scans, or the body movement information, and displays the body movement information in association with the progress of the scan in the display region of the at least one.

According to the aspect of the present invention, in a case where a plurality of scan tasks are continuously performed, it is possible to provide a progress status of each scan and a body movement status of a subject during the progress to a user in a form that is understandable at a glance. As a result, it is possible to grasp at a glance a body movement occurring while the plurality of scans are performed. In addition, it is possible to prevent inappropriate imaging position determination or scan parameter setting in advance, and it is possible to perform main imaging based on imaging positions and scan parameters that are appropriately set.

### BRIEF DESCRIPTION OF THE DRAWINGS

Fig. 1 is a diagram showing an overall overview of an MRI apparatus.
Fig. 2 is a diagram showing an example of disposition of a body movement detection unit in the MRI apparatus.
Fig. 3 is a diagram showing an example of body movement information.
Fig. 4 is a flowchart showing an embodiment of an operation of the MRI apparatus.
Fig. 5 is a diagram for describing a display region of a display screen of Embodiment 1.
Fig. 6 is a diagram showing a display example of the body movement information according to Embodiment 1.
Fig. 7 is a diagram showing an example of a mark indicating the body movement information.
Fig. 8 is a diagram showing a display example of the body movement information in a scan information display region.
Figs. 9A and 9B are diagrams showing display examples of the body movement information according to Embodiment 2, in which Fig. 9A shows a case where there is one type of body movement information, and Fig. 9B shows a case where there are two types of body movement information.
Fig. 10 is a diagram showing a display example of Embodiment 2.
Fig. 11 is a diagram showing an example of a display unit of Embodiment 3.
Figs. 12A and 12B are diagrams showing display screen examples of Embodiment 3, in which Fig. 12A shows a display example in a case where there is no problematic body movement, and Fig. 12B shows a display example in a case where a body movement has occurred.

### DESCRIPTION OF THE PREFERRED EMBODIMENTS

First, an overview of an MRI apparatus to which the present invention is applied will be described.

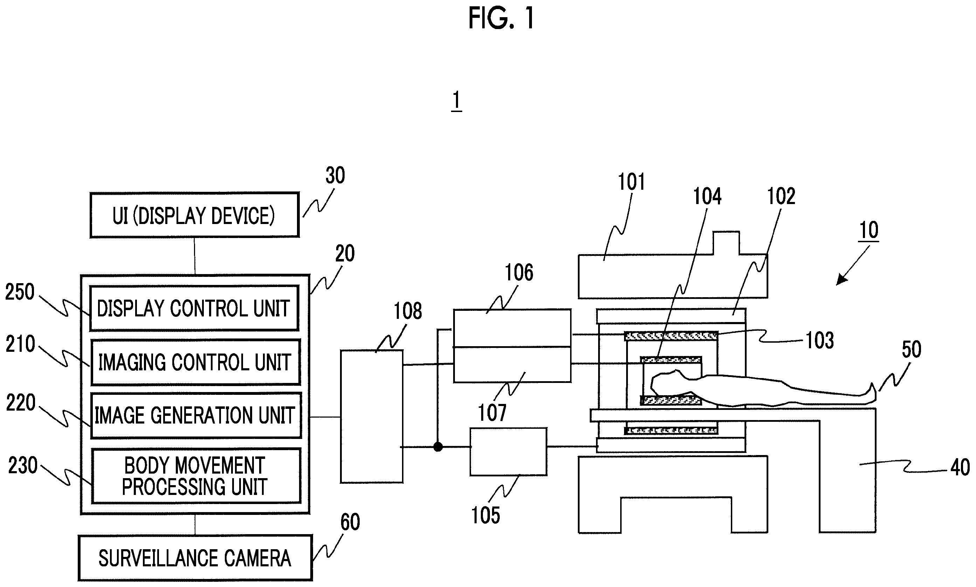

As shown in Fig. 1, the MRI apparatus is roughly divided into an imaging unit 10 and a processor 20 that performs various controls and calculations. Main elements constituting the imaging unit 10 are housed in a gantry that provides an examination space. A gantry 100 is installed in an examination room shielded from electromagnetic waves as shown in Fig. 2, and the processor 20 and a console 200 acting as a user interface are installed in an operation room separate from the examination room.

The imaging unit 10 induces nuclear magnetic resonance in nuclei (usually protons) of atoms that constitute a tissue of a subject, and collects nuclear magnetic resonance signals (NMR signals) generated thereby from the subject. Hereinafter, the nuclear magnetic resonance signal is also simply referred to as a signal or an echo signal.

A configuration of the imaging unit 10 is similar to that of a known MRI apparatus, and comprises a static magnetic field magnet 101 that generates a uniform magnetic field (static magnetic field) in the examination space in which a subject 50 is placed, a gradient magnetic field coil 102 that applies a gradient magnetic field to the static magnetic field, an RF transmission coil 103 that applies a high-frequency magnetic field to excite the nuclei of the atoms constituting the tissue of the subject, and an RF reception coil 104 that receives an NMR signal generated by the subject, in which the gradient magnetic field coil 102, the RF transmission coil 103, and the RF reception coil 104 are connected to a gradient magnetic field power supply 105, a transmitter 106, and a receiver 107, respectively. Operations of the gradient magnetic field power supply 105, the transmitter 106, and the receiver 107 are controlled by a sequencer 108. The sequencer 108 determines a pulse sequence for each scan using a set pulse sequence type and imaging conditions such as imaging parameters, and controls each component of the imaging unit 10 to operate in accordance with the determined pulse sequence and collect an echo signal (k-space data) necessary for image reconstruction. Since functions and operations of each component in a case where the imaging unit 10 collects the k-space data are similar to those of a general MRI apparatus, detailed description thereof will be omitted here.

The static magnetic field magnet 101, the gradient magnetic field coil 102, and the RF transmission coil 103 are housed in the gantry, and the subject 50 is positioned in the examination space in the gantry in a state of being laid on a bed device 40 with the RF reception coil 104 attached to an examination area.

The processor 20 has a function as a control unit that controls imaging via the sequencer 108 and that controls operations of the entire apparatus, and a function as a calculation unit that reconstructs an image of the subject using the echo signal collected by the imaging unit 10 or performs a calculation such as correction on the k-space data before reconstruction or an image after reconstruction. The processor 20 of the present embodiment has a function of collecting and processing body movement information generated in the subject 50 during an examination, for example, a magnitude and a duration of a body movement, and associating the body movement information with a scan in progress (imaging) to be presented to a user, in addition to the functions of the control unit and the calculation unit described above.

In order to realize the above functions, the processor 20 comprises an imaging control unit 210 that controls imaging via the sequencer 108, an image generation unit 220 that reconstructs an image of the subject using the k-space data collected by the imaging unit 10, a body movement processing unit 230 that collects body movement information from a unit that detects a body movement occurring in the subject 50 during an examination and performs various types of processing related to the body movement, and a display control unit 250 that controls a display device or the like to present the image or the body movement information to the user.

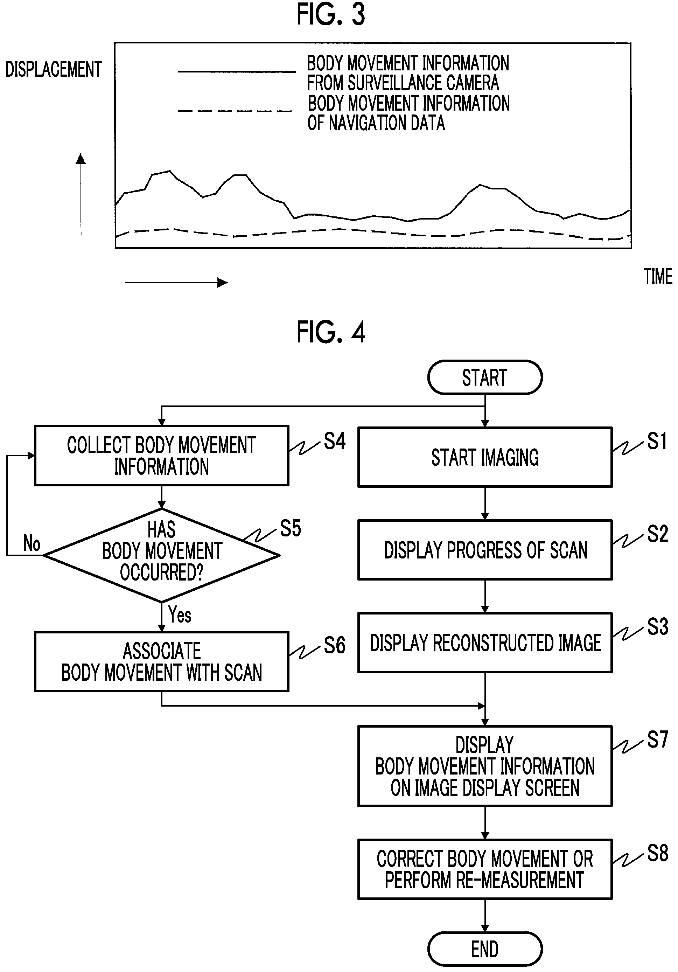

In a case where a surveillance camera or other optical detection units for detecting the movement of the subject are attached to the MRI apparatus, the body movement processing unit 230 inputs the movement of the subject acquired by the detection units, and collects body movement information such as an area where a body movement occurs, a magnitude of the body movement, a temporal change in displacement, and a duration. As a result, for example, information regarding the body movement as shown in Fig. 3 is obtained. As a method of obtaining body movement information from a video of the surveillance camera, a method of analyzing the video for each frame by optical flow or the like to acquire displacements or motion vectors of feature points of the subject included in the video or a coil attached to the subject is known, and in the present embodiment, the body movement information is collected by the known method.

In addition, it is also possible to collect navigator data using the imaging unit 10 to detect the displacement of the subject and to collect the body movement information using the navigator data, in addition to the external body movement detection unit such as a surveillance camera 80. The navigator data is a set of NMR signals (navigator echoes) generated from the subject separately from the signal for generating the image of the subject, and the body movement processing unit 230 collects the body movement information of the subject by analyzing time-series variations in the navigator echoes. A pulse sequence for generating the navigator echoes and a method of collecting the body movement information using the navigator echoes are known (for example, JP2021-183031A), and such a known method can also be adopted in the present embodiment. Although detailed description will be omitted, for example, a method of selecting and exciting a region including a diaphragm, acquiring a displacement of the diaphragm from an image reconstructed from navigator data collected from the region, and obtaining a periodic movement can be adopted.

The display control unit 250 displays the body movement information collected by the body movement processing unit 230 on the display device in association with the progress of the scan. In a case where a display screen is divided into a plurality of display regions by display items, the body movement information is displayed in any one of the display regions.

Functions of the imaging control unit 210 and the image generation unit 220 are similar to those of imaging control and image generation of a general MRI apparatus. However, in a case where the body movement information is acquired from the navigator data as described above, the imaging control unit 210 controls the imaging unit 10 by adding a sequence for acquiring the navigator echoes. In addition, depending on the body movement information collected by the body movement processing unit 230, the imaging control unit 210 may perform control to cause the imaging unit 10 to perform re-measurement, or the image generation unit 220 may perform processing such as body movement correction on the collected k-space data or the image reconstructed from the k-space data.

The functions of the processor 20 described above can be implemented by one or a plurality of computers comprising a CPU and a memory. However, some of the functions implemented by the processor 20 may be implemented by a programmable IC such as an ASIC or a FPGA.

Further connected to the MRI apparatus as additional devices are a display device 30 for displaying the image generated by the image generation unit 220, the body movement information collected or processed by the body movement processing unit 230, a GUI for the user to set conditions and input commands to the apparatus, other output devices (not shown), and a storage device (external storage device 60 or the like) for storing the image, the body movement information, and the like. The display device 30 can be installed at one or a plurality of locations such as the console 200 for the user to operate the apparatus and a front surface of the gantry 100. In the example shown in Fig. 2, the display device 30 is installed at two or more locations on a gantry side and a console side, so that the image and the body movement information can be checked from both an inside of the examination room and the operation room.

In addition, in the MRI apparatus, the surveillance camera 80 for monitoring the subject 50 is installed at one or a plurality of locations in the vicinity of the gantry. A video from the surveillance camera 80 is sent to the body movement processing unit 230. As described above, the body movement processing unit 230 collects the body movement information by analyzing the video for each frame. In the example shown in Fig. 2, two surveillance cameras 80 are installed at both ends of the gantry to look into the examination room. This configuration allows comprehensive detection of the movement of the subject in the examination room.

The operation of the MRI apparatus in the above-described configuration will be described. Fig. 4 shows a flow of the operation.

First, under the control of the imaging control unit 210, the imaging is started according to a preset examination protocol (S1).

Typically, an MRI examination is performed according to an examination protocol in which a type of imaging (scan task) performed by the imaging unit 10, imaging conditions of each scan, an order in which scans are performed, and the like are determined in advance. The scan tasks included in the protocol vary depending on an imaging area or an imaging purpose, but includes, for example, scanogram imaging (multiple cross-sectional imaging) for determining an imaging position, and main imaging of one or a plurality of cross sections, for example, one or more of T1-weighted imaging, FLAIR (in particular, in a case of imaging a brain), diffusion-weighted imaging (DWI), and T2*-weighted imaging.

The examination protocol can be set in advance by default or can be set or changed by the user through the display control unit 250, according to the purpose of the examination. For example, the display device 30 displays a list of scan tasks included in the set examination protocol and imaging conditions for each of the scan tasks, and accepts changes made by the user.

In a case where the processor 20 reads the examination protocol consisting of a plurality of scan tasks designated by the user, the imaging control unit 210 starts imaging in accordance with the examination protocol.

In a case where the examination is started according to the set examination protocol, the display control unit 250 acquires time information of the progress of the scan from the imaging control unit 210 and displays a progress status of each scan included in the examination protocol on the display device 30 (S2). For example, in the display region in which the list of scan tasks is displayed, the scan task being performed may be displayed in an identifiable manner, for example, by changing brightness or display color, and the progress status of the scan task may be shown by time.

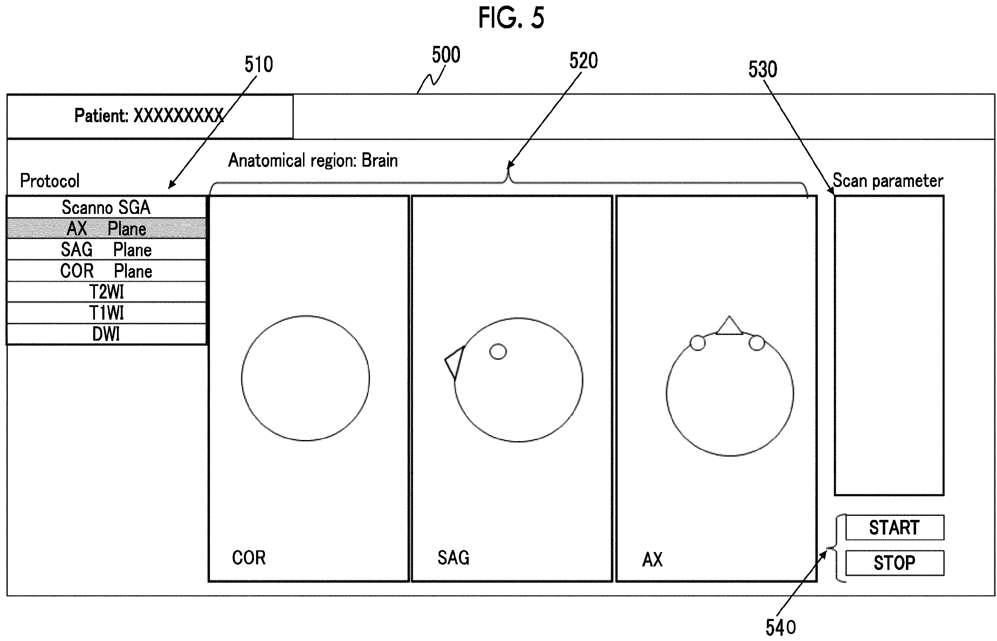

Fig. 5 shows an example of the display screen. In this example, the display screen 500 is provided with a scan information display region (first region) 510 that shows the list of scan tasks and the progress status, a region (second region) 520 that displays an image obtained by the scan, a region 530 that displays a scan parameter (imaging parameter), a region 540 that displays a GUI such as a button for inputting a user command such as start or end of imaging, and the like. The first region 510 can also display scan tasks included in the protocol and display the scan currently in progress in an identifiable manner, for example, by displaying the scan currently in progress in the list of scan tasks in a different color or brightness from other scans, or with a frame, an arrow, or the like.

As each scan progresses, in a case where image-reconstructable data (k-space data) is collected, the image generation unit 220 reconstructs an image using the k-space data (S3). The display control unit 250 displays the reconstructed image in the image display region 520 of the display device 30.

On the other hand, the body movement processing unit 230 collects body movement information from detection information from the surveillance camera 80 or from the navigator data (S4). As shown in Fig. 3, primary information of a body movement obtained by the body movement processing unit 230 from the body movement detection unit is a displacement of a specific position such as an examination area of the subject along a time axis, and the body movement processing unit 230 processes the primary information, compares the primary information with a preset displacement threshold, determines whether or not a body movement that affects the imaging has occurred (S5), and obtains an occurrence time and a duration of the body movement that affects the imaging as secondary information. In a case where the body movement information at a plurality of locations (a plurality of feature points) is acquired using the video from the surveillance camera 80, information of a position where the body movement occurred may be included. In addition, by using the body movement information collected from the navigator data and the body movement information collected from the video of the camera, or by setting two or more thresholds for a displacement range, it is possible to distinguish between movements such as respiratory movements, which have a relatively small fluctuation range, and other sudden body movements. The body movement processing unit 230 passes at least one body movement information of the acquired primary information or the secondary information obtained by analyzing the primary information to the display control unit 250.

The display control unit 250 associates the two with each other using time information included in the body movement information and the time information of the progress of the scan, and displays the time information in at least one of the image display region 520 or the scan information display region 510 of the display device 30 such that a relationship between the scan and the body movement can be seen (S6, S7). As a display aspect of the body movement information associated with the progress of the scan, (A) displaying the occurrence time of the body movement and the magnitude of the body movement as text information, (B) displaying a graph of the displacement of the body movement, (C) displaying a mark indicating the occurrence of the body movement, or a combination thereof can be adopted. A specific aspect of the display will be described in the following embodiments.

The image generation unit 220 performs necessary body movement correction based on the relationship between the body movement scan and the body movement. Alternatively, the imaging control unit 210 performs control to perform re-measurement for the scan that is greatly affected by the body movement (S8).

With the MRI apparatus according to the present embodiment, in a case where a plurality of scans are continuously performed, the progress of each scan and the body movement in the progress of the scan are associated with each other and presented to the user in an easily understandable manner, so that the user can take an appropriate response to eliminate the effect of the body movement. In addition, in a case of the scanogram imaging in which a plurality of cross sections are continuously imaged, it is possible to reduce the effect of the body movement occurring during the scan of one cross section on position determination of the next cross section and imaging of the cross section by a different scan, and to prevent positional deviations in the scans performed on each cross section.

Hereinafter, a specific aspect in which the display control unit 250 presents the body movement and the progress of the scan in association with each other will be described.

### Embodiment 1

In the present embodiment, a case where a scan for imaging a plurality of cross sections is the main imaging, for example, imaging three cross sections of AX, SAG, and COR in the same scan type will be described as an example, but the scan itself may be a plurality of different cross sections, and the number of cross sections may be small. In addition, the scan may be positioning imaging instead of the main imaging. Furthermore, in the present embodiment, while a positioning scan of three different cross sections is in progress, the body movement information collected in real time by the body movement processing unit 230 is displayed in the image display region in which the image obtained in each scan is displayed. In addition, it is assumed that the body movement processing unit 230 collects the body movement information using the video of the surveillance camera 80, as an example.

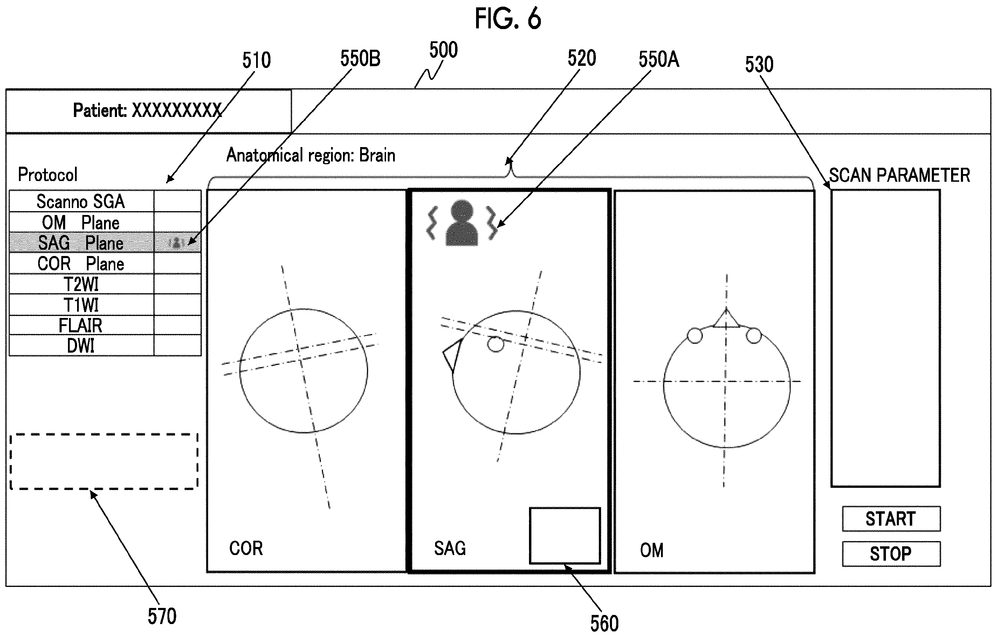

Fig. 6 shows an example of a display screen of the present embodiment. In the present embodiment, it is assumed that the display screen of the display device 30 is provided with the scan information display region 510, the image display region 520, and the scan parameter display region 530, as in Fig. 5.

As shown in Fig. 6, in this example, a case where the body movement that affects the image is detected while the SAG imaging is in progress shown, and a mark 550A indicating the occurrence of the body movement is shown in the image display region 520 in which the image obtained by the SAG imaging is displayed. The mark 550A has a shape resembling a part of the human body, in this case, including the head as the imaging area, and for example, marks different for each examination area can be stored in advance in the memory or the storage device in the processor 20 and can be read and displayed by the display control unit 250.

In a case where information indicating that the body movement has occurred in the vicinity of the imaging area is acquired as the body movement information, the body movement processing unit 230 displays the mark 550A in a background portion that does not overlap with a portion of the image of the subject in the image display region in which the image obtained by the scan performed at a body movement occurrence time is displayed, and notifies the user that the image obtained by the scan is affected by the body movement, for example, that there is a high probability that a positional deviation has occurred due to the body movement.

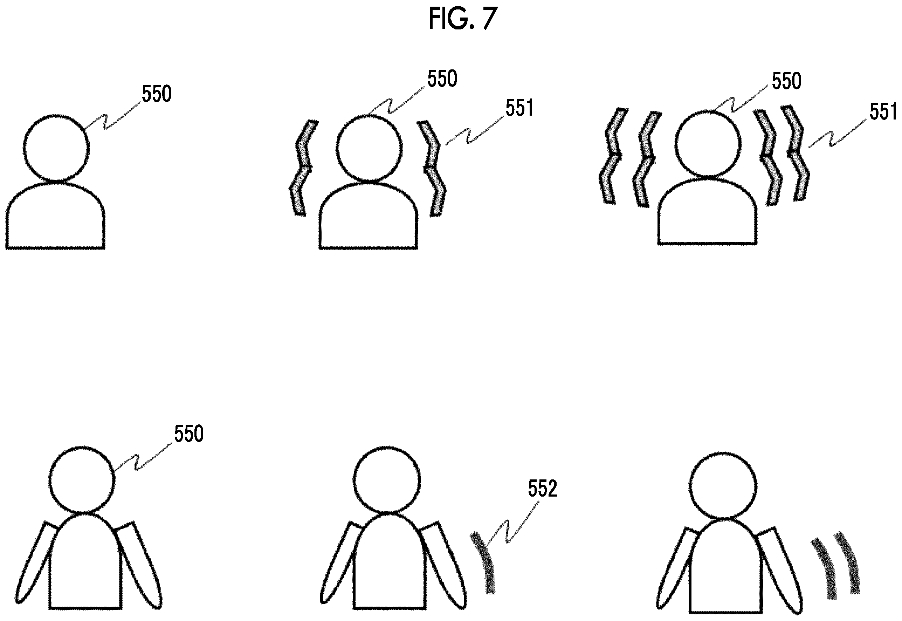

Regarding the mark indicating the occurrence of the body movement, the mark may be changed according to the magnitude of the body movement. For example, as shown on an upper side of Fig. 7, linear marks 551 of which the number increases according to the magnitude of the body movement may be added to an outside of the mark 550 that imitates the human body. In Fig. 6, a state in which the marks 551 are displayed is shown. As a result, the user can estimate the magnitude of the effect of the body movement on the image and can take an appropriate response. For example, in a case where the body movement is large, the measurement is repeated and an imaging cross section is determined by an image obtained in a case where there is no body movement.

In addition, as shown on a lower side of Fig. 7, linear marks 552 may be added to an area where the body movement occurs in the mark 550 indicating the occurrence of the body movement, so that the occurrence area can be identified. For example, in a case where the imaging area is the brain, movements of the head have a large effect on the image even in a case where the movements are small, but movements of an arm or a finger have a small effect on the image. Therefore, depending on the area where the body movement occurred, the user can determine whether or not to determine the imaging cross section using the image obtained by the scan in which the body movement has occurred, as it is.

Instead of the mark or together with the mark, more detailed body movement information, for example, information such as a body movement occurrence time, a duration, a magnitude of the body movement (magnitude classified into "large", "medium", "small", or the like by a threshold), and an area where a body movement occurred may be displayed in text 560 in the background portion of the image display region. Only the text may be displayed, but by using the text in combination with a mark, the user can grasp the occurrence of the body movement at a glance, and can respond quickly.

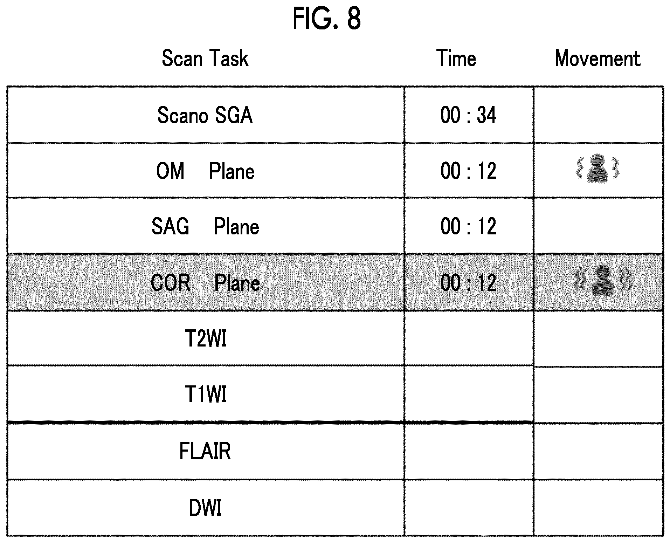

In addition, the same mark 550B as the mark 550A displayed in the image display region may be displayed for the corresponding scan displayed in the scan information display region 510. Fig. 8 shows only the scan information display region 510 in an enlarged manner. In this example, a column indicating the scan time of the list of scans and a column indicating the body movement information are provided, and the mark 550B is displayed for the scan in which the body movement is detected. By the displaying in this scan information display region, it is possible to notify of the occurrence of the body movement even before the reconstructed image is displayed, that is, even during the collection of the k-space data, allowing the user to respond more quickly.

According to the present embodiment, in a case where a plurality of cross sections are imaged, in a process in which a scan of different cross sections is in progress, the body movement information associated with the progress status can be provided to the user in real time. As a result, the user can grasp information about which cross section is being imaged while the body movement occurs. In addition, in a case where the imaging of the plurality of cross sections is the positioning imaging, it is possible to perform re-measurement more quickly for diagnosis using these images, thereby reducing the probability of inappropriate diagnosis, and reducing the need for re-imaging.

The display screen may be provided with a region 570 in which the body movement information obtained as the primary information is displayed, and the like, and this region may also display the body movement information and the progress of the scan in association with each other. This display form will be described in the embodiment described below.

In addition, according to the present embodiment, by pasting the body movement information into the display region of the image, it is possible to clearly grasp whether or not the body movement has occurred in the actually acquired image without referring to other display information or the like, enabling the user to respond quickly. In particular, by indicating the presence or absence and the magnitude (degree) of the body movement with the mark, it is possible to intuitively and easily grasp the occurrence of the body movement.

In the above description, the present embodiment has been described using the case where three cross sections are imaged in one scan as an example. However, for a plurality of scans included in the main imaging, it is common to image the same cross section or a plurality of cross sections and to refer to each cross-sectional image for diagnosis. In such a case, in a case where the body movement occurs in any of the plurality of images to be referred to, the plurality of images may not be used as the information to be referred to, or accuracy may be reduced even in a case in which the plurality of images are used. The present embodiment can also be applied to scans that require a relationship between the images, and thus, it is possible to grasp images with low reliability, and to suppress a decrease in diagnostic accuracy.

### Embodiment 2

### Display Examples of Body Movement Graph

In the present embodiment, a progress status of a scan associated with body movement information is displayed on a graph showing the body movement information.

A display device that displays the graph showing the body movement information is not particularly limited. However, in general, in an MRI examination or the like, a dedicated display device may be provided to display vital information such as an electrocardiographic waveform, a photoplethysmography signal waveform (pulse rate), and a respiratory rate of the subject during the examination. The body movement information graph targeted by the present embodiment can be displayed on such a dedicated display device, but may also be displayed together with the scan information and the image on at least one of the console 200 of the MRI apparatus or the display device 30 installed on an outer surface of the gantry. For example, the body movement information graph is displayed in the display region 570 surrounded by a dotted line in Fig. 6.

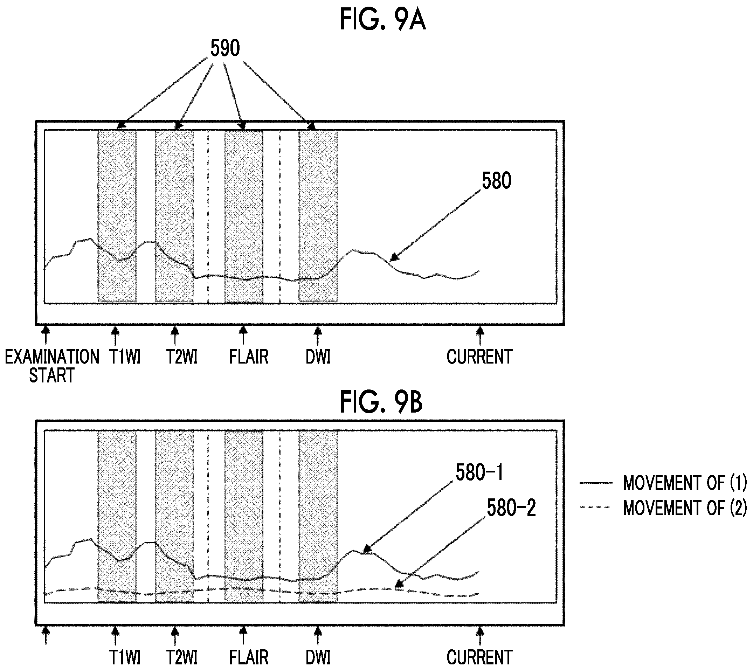

Display examples of the body movement graph in which the body movement information and the progress of the scan are associated with each other are shown in Figs. 9A and 9B. In Figs. 9A and 9B, a horizontal axis represents time, and a vertical axis represents a magnitude of a displacement.

In Figs. 9A and 9B, for example, a displacement of a predetermined one or plurality of positions (feature points) obtained by analyzing the video from the surveillance camera 80 is displayed as a graph 580. Fig. 9A shows the displacement of one position, and Fig. 9B shows graphs 580-1 and 580-2 of displacements of two positions. In addition, instead of or in addition to displaying the graph of the displacements of the two positions, the body movement information obtained by a different detection unit may be displayed. For example, the body movement information obtained from the surveillance camera 80 and the body movement information acquired by the navigator echo may be included in a single display. By displaying the information from the surveillance camera and the information from the navigator echoes together, for example, it is possible to distinguish between and grasp a periodic movement detected by the navigator echoes and a sudden movement obtained from the surveillance camera.

In a case where a dedicated display device is used to monitor the body movement and the progress of the scan, it is also possible to display body movement information from each of a plurality of positions or a plurality of detection units.

In addition, in the present embodiment, the time information of the progress of the scan received from the imaging control unit 210 is displayed in a superimposed manner together with the graph 580 of the body movement information.

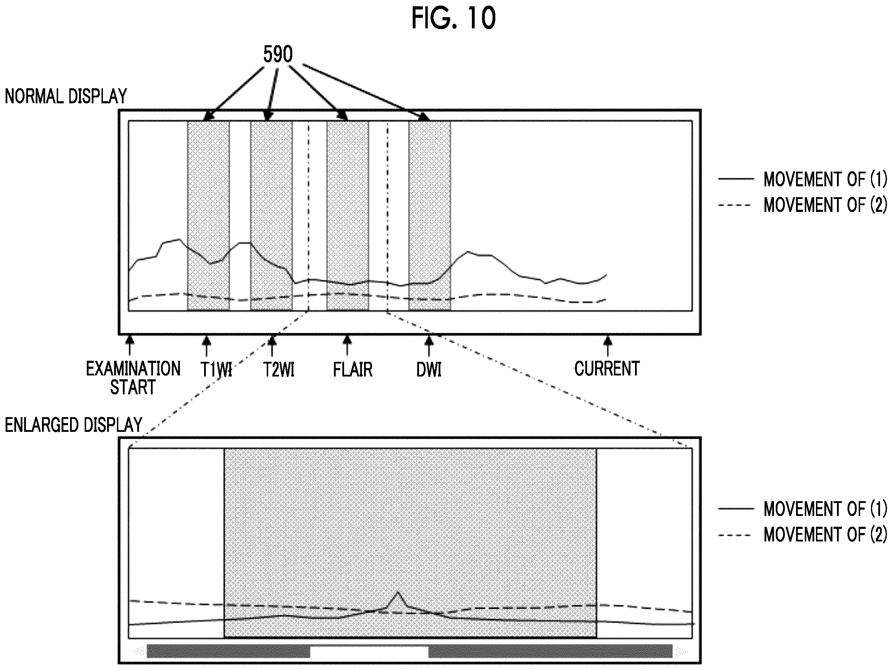

In the example shown in Fig. 9, in a case where a scan is started, a band 590 corresponding to the scan is displayed on the graph in a superimposed manner with a predetermined transparency. For example, as shown in Fig. 10, each scan and the associated body movement information can be displayed in an enlarged manner, and detailed body movement information such as a point in time in the scan at which the body movement that affects the scan occurs can be confirmed. In displaying of the scan progress status using a band, a configuration may be adopted in which a width of the band 590 is extended as each scan progresses, and the extension is ended at a point in time when the scan is completed. Thereafter, in a case where the next scan is started, a band is displayed in the same manner from the start time point, and a width of the band is extended until the scan is completed. A color or brightness of the band may be different for the completed scan and the scan in progress.

By checking the graph, the user can immediately grasp whether or not the body movement occurs during the scan currently in progress.

According to the present embodiment, by displaying the progress of the scan in a manner superimposed on the body movement information obtained from the surveillance camera that monitors the body movement of the subject or from the navigator data, it is easy to grasp in which scan the body movement occurred and at what point in time during the scan the body movement occurred. In addition, in the present embodiment, since a plurality of pieces of body movement information, for example, body movement information of different areas or body movement information obtained from different body movement detection units are displayed in a single display region, it becomes easy to grasp whether or not body movement that is a problem for the examination area occurs.

### Embodiment 3

### Presentation to Subject

The present embodiment is characterized by a configuration that allows the subject during an examination to check a display content in which the body movement information and the progress of the scan are associated with each other.

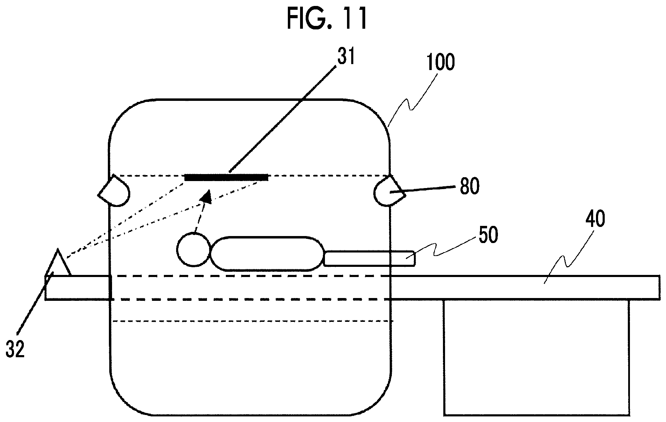

Specifically, as shown in Fig. 11, a display unit 31 may be installed at a position in the examination space inside the gantry at which the subject during examination can be visually recognized, so that the subject 50 can check the progress of the scan and the body movement.

In the example shown in Fig. 11, a configuration is adopted in which an optical video display unit 32, such as a projector, is installed at an end part of the bed device 40 located outside the gantry 100, and a video is projected from the video display unit onto a ceiling surface (display unit 31) of the gantry 100. In addition, a configuration may be adopted in which a video projected by a projector from an outside of the gantry is reflected by a mirror and projected onto a reflector disposed near the head of the subject.

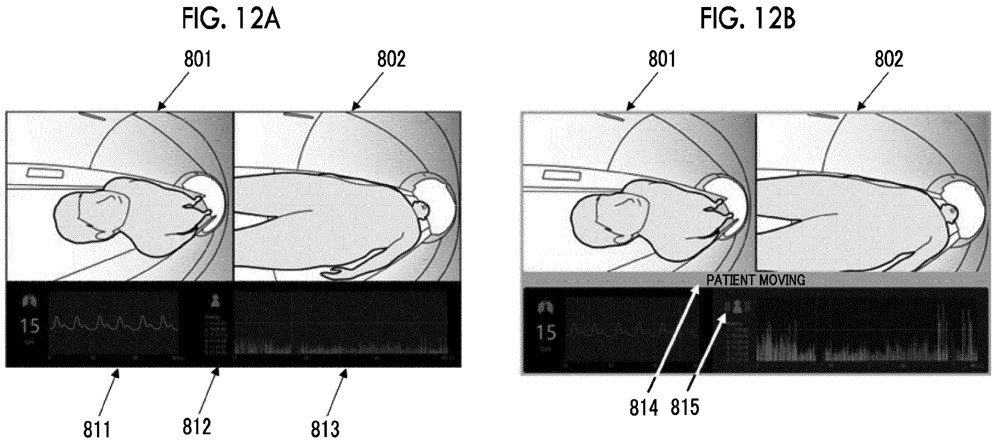

Figs. 12A and 12B show display examples displayed on the display unit 31. This example is a display example in a case where the surveillance camera 80 is provided at both ends of an opening of the gantry 100 as shown in Fig. 2, and vital information 811 such as heart rate information and respiratory information, scan information 812, and body movement information 813 are displayed together with videos 801 and 802 of the two surveillance cameras 80. Such a display is implemented by the display control unit 250 receiving vital information such as an electrocardiogram or a balloon that detects respiratory movements directly from the electrocardiogram or via the body movement processing unit 230, and the body movement processing unit 230 controlling the display device (here, the video display unit 32) to display the vital information together with the videos 801 and 802 acquired from the surveillance cameras. Fig. 12B on the right side shows a state in which there is no problematic body movement, but in a case where the subject has moved, a mark 815 (a mark indicating a body movement outside a mark imitating the human body) indicating the body movement is displayed together with text information "PATIENT MOVING" 814 indicating that the body movement has occurred.

The subject 50 during an examination can check his/her own state including the body movement during the examination from the display, and can actively participate in the examination, for example, by reducing the body movement, or can quickly inform an examiner of the abnormality during the examination.

By adopting the display aspect of Embodiment 1 or Embodiment 2 described above, substantially the same content as that displayed for the subject 50 can also be displayed on the display device 30 that can be checked by an examination technician or a doctor.

According to the present embodiment, it is possible to present the progress of the scan and the body movement information in association with each other in a state that is visible to the user 50, and thus, it is possible to motivate the subject 50 to actively cooperate with the examination, which leads to a reduction in anxiety of the subject 50.

As described above, according to the present invention, an interface between the MRI apparatus and the user including the examination technician, the doctor, the subject, and the like in the MRI apparatus is improved, and the examination can be advanced while each user checks an examination situation including the body movement of the subject, thereby improving the quality of the examination.

**In** addition, according to the present invention, in particular, in a case of imaging a plurality of cross sections that are related to each other, it is possible to grasp in real time which cross section is being scanned when problematic body movement occurs, and it is possible to minimize the effect of the result of the scan in which the body movement occurs on the scan of the other cross sections, thereby reducing the number of scans that need to be redone, and as a result, eliminating unnecessary extensions of examination times.

### Explanation of References

10: imaging unit
20: processor
30: display device
40: bed device
60: external storage device
80: surveillance camera
100: gantry
200: console
210: imaging control unit
220: image generation unit
230: body movement processing unit
250: display control unit

## Claims

1. A magnetic resonance imaging apparatus comprising:
an imaging unit (10) that performs a plurality of scan tasks including imaging of different cross sections and collects a nuclear magnetic resonance signal generated from a subject for each of scans; and
a processor (20) including
an image generation unit (220) that generates an image of each of the scans using the nuclear magnetic resonance signal,
a body movement processing unit (230) that collects and processes body movement information related to a body movement of the subject, and
a display control unit (250) that displays the image and the body movement information on a display device (30),
wherein the processor associates progress of the scan with the body movement information including a magnitude and an occurrence time of the body movement collected by the body movement processing unit, and
the display control unit sets a display region on the display device to display at least one of the plurality of scan tasks, the image obtained in each of the scans, or the body movement information, and displays the body movement information in association with the progress of the scan in the display region of the at least one.

2. The magnetic resonance imaging apparatus according to claim 1,
wherein the plurality of scan tasks include a plurality of scans each for imaging different cross sections of the subject, and
the display control unit (250) adds, in a case where a body movement occurs while the plurality of scan are performed, display information indicating a scan of a cross section in which the body movement has occurred, to a display region of an image of the different cross sections sequentially acquired in the scans.

3. The magnetic resonance imaging apparatus according to claim 2,
wherein the different cross sections include at least two cross sections of an AX cross section, a COR cross section, or a SAG cross section.

4. The magnetic resonance imaging apparatus according to claim 2 or 3,
wherein the plurality of scan tasks include a plurality of positioning scans each for imaging different cross sections of the subject.

5. The magnetic resonance imaging apparatus according to claim 1,
wherein the display of the body movement information associated with the progress of the scan is text information describing the body movement information, and
the display control unit (250) displays the text information in the display region of the at least one.

6. The magnetic resonance imaging apparatus according to claim 1,
wherein the display of the body movement information associated with the progress of the scan is an icon image indicating the body movement, and
the display control unit (250) changes a form of the icon image according to the magnitude of the body movement.

7. The magnetic resonance imaging apparatus according to claim 1,
wherein the display control unit (250) displays the body movement information as a graph in which a vertical axis represents the magnitude of the body movement and a horizontal axis represents time in the display region displaying the body movement information, and superimposes and displays a range in which each scan is in progress on the graph.

8. The magnetic resonance imaging apparatus according to claim 7,
wherein the display control unit (250) displays a range in which the plurality of scan tasks are in progress on the graph in an enlarged manner.

9. The magnetic resonance imaging apparatus according to claim 1,
wherein the display device (30) includes a first display device and a second display device which are placed at different positions, and
the display control unit (250) displays the image and scan information on the first display device, displays the body movement information on the second display device, and displays the body movement information associated with the progress of the scan in each of a display region of the image or the scan information of the first display device and a display region of the body movement information of the second display device.

10. The magnetic resonance imaging apparatus according to claim 9,
wherein the second display device is disposed in an examination space in which the subject is placed and is visible from the subject.

11. The magnetic resonance imaging apparatus according to claim 1 or 7,
wherein the body movement information includes a plurality of pieces of body movement information that are different in any of a type of the body movement, a generation area of the body movement, or a unit for acquiring the body movement information, and
the display control unit (250) displays the plurality of pieces of body movement information in parallel or in a superimposed manner.

12. The magnetic resonance imaging apparatus according to claim 11,
wherein the plurality of pieces of body movement information include body movement information acquired from a body movement detection device provided in the magnetic resonance imaging apparatus, and body movement information acquired from a navigator echo collected by the imaging unit, or wherein the plurality of pieces of body movement information include a periodic movement of the subject, and an irregular body movement of the subject.

13. A display method for presenting scan information and body movement information to a user during magnetic resonance imaging including a plurality of scan tasks, the display method comprising:
displaying, on a display device (30), a screen including a first display region in which the plurality of scan tasks are displayed and a second display region in which an image acquired by imaging in each of the scan tasks is displayed; and
associating each of the scan tasks with a body movement of a subject during imaging that occurs in a scan, and displaying body movement information associated with progress of the scan and the progress of the scan in association with each other in at least one of the first display region or the second display region.

14. The display method according to claim 13,
wherein the plurality of scan tasks include a plurality of positioning scans for imaging different cross sections of the subject, and
in a case where the body movement occurs while the plurality of positioning scans are performed, display information indicating a scan of a cross section in which the body movement has occurred is added to the first display region or the second display region.

15. The display method according to claim 13,
wherein the body movement information includes a plurality of pieces of body movement information that are different in any of a type of the body movement, a generation area of the body movement, or a unit for acquiring the body movement information, and
the plurality of pieces of body movement information are displayed in parallel or in a superimposed manner.
